# EUROPÄISCHE PATENTSCHRIFT

(11) **EP 4 048 202 B1**
(45) Veröffentlichungstag und Bekanntmachung des Hinweises auf die Patenterteilung: **03.12.2025**
(21) Anmeldenummer: 20797399.1
(22) Anmeldetag: 15.10.2020
(51) Int. Cl.: A61F 2/16

(54) **INJEKTOR FÜR EIN EINFÜHREN EINER INTRAOKULARLINSE**
INJECTOR FOR INSERTING AN INTRAOCULAR LENS
INJECTEUR SERVANT À L'INSERTION DE LENTILLE INTRAOCULAIRE

(30) Priorität: 21.10.2019 DE 102019128370
(43) Veröffentlichungstag der Anmeldung: 31.08.2022
(73) Patentinhaber: Carl Zeiss Meditec AG, 07745 Jena (DE)
(72) Erfinder: RAQUIN, Vincent, 17000 La Rochelle (FR); PANKIN, Dmitry, 12623 Berlin (DE); RATHERT, Brian, 45659 Recklinghausen (DE)
(74) Vertreter: PATERIS Patentanwälte PartmbB
(86) Internationale Anmeldenummer: PCT/EP2020/079021
(87) Internationale Veröffentlichungsnummer: WO 2021/078623

(56) Entgegenhaltungen:
- WO-A2-2008/036597
- DE-A1- 102013 105 184
- GB-A- 2 493 017
- US-A1- 2008 147 080

## Beschreibung

Die Erfindung betrifft einen Injektor für ein Einführen einer Intraokularlinse in den Kapselsack eines Auges.

Eine hydrophile Intraokularlinse ist für ihre Lagerstabilität in einem wässrigen Medium zu lagern. Ein Injektor zum Einführen der Intraokularlinse in den Kapselsack weist eine Beschichtung auf, die ein Gleiten der Intraokularlinse in dem Injektor erleichtert. Dies ist besonders relevant an Stellen des Injektors, an denen die Intraokularlinse besonders stark komprimiert ist. Weil sich die Beschichtung bei Kontakt mit Wasser auflöst, ist der Injektor im Gegensatz zu der Intraokularlinse trocken zu lagern. Daher wird herkömmlich die Intraokularlinse in einer ersten sterilen Verpackung gelagert und der Injektor wird in einer zweiten sterilen Verpackung gelagert. Vor dem Einführen der Intraokularlinse in den Kapselsack ist die Intraokularlinse von Hand in den Injektor einzusetzen. Dies birgt jedoch ein Risiko, dass bei dem Einsetzen der Intraokularlinse in den Injektor eine Kontamination der Intraokularlinse und/oder des Injektors erfolgt.

WO 2008/036597 A2 beschreibt eine Versendungsvorrichtung für ein medizinisches Gerät.

Aufgabe der Erfindung ist es daher, einen Injektor mit einer Intraokularlinse zu schaffen, bei dem die vorgenannten Probleme überkommen sind.

Gemäß einer ersten Ausführungsform des erfindungsgemäßen Injektors weist der Injektor einen Zylinder, der einen ersten Zylinderabschnitt und einen zweiten Zylinderabschnitt aufweist, die in Längsrichtung des Zylinders nebeneinander angeordnet sind, eine Aufnahme, die zwischen den beiden Zylinderabschnitten angeordnet ist, und ein Einsetzbauteil auf, das in der Aufnahme angeordnet ist sowie eine Intraokularlinse, eine Aufbewahrungsflüssigkeit, ein Gehäuse, eine innerhalb des Gehäuses angeordnete Faltkammer, in der die Intraokularlinse von der Aufbewahrungsflüssigkeit umgeben angeordnet ist, und einen Deckel aufweist, der die Faltkammer gegenüber dem ersten Zylinderabschnitt abdichtet sowie an dem Gehäuse befestigt ist, wobei das Einsetzbauteil eingerichtet ist, mittels einer Bewegung des Gehäuses relativ zu der Faltkammer die Faltkammer von dem Deckel freizulegen und somit zu öffnen sowie die Faltkammer zu deformieren und dadurch die Intraokularlinse zu falten.

Gemäß einer zweiten Ausführungsform des erfindungsgemäßen Injektors weist der Injektor einen Zylinder, der einen ersten Zylinderabschnitt und einen zweiten Zylinderabschnitt aufweist, die in Längsrichtung des Zylinders nebeneinander angeordnet sind, eine Aufnahme, die zwischen den beiden Zylinderabschnitten angeordnet ist, ein Einsetzbauteil, das in der Aufnahme angeordnet ist sowie eine Intraokularlinse, eine Aufbewahrungsflüssigkeit, ein Gehäuse, eine innerhalb des Gehäuses angeordnete Faltkammer, in der die Intraokularlinse von der Aufbewahrungsflüssigkeit umgeben angeordnet ist, einen Deckel, der die Faltkammer gegenüber dem ersten Zylinderabschnitt abdichtet, und ein erstes Bauteil aufweist, an dem der Deckel angeordnet ist, und ein zweites Bauteil auf, wobei der Injektor eingerichtet ist, mittels einer Bewegung des ersten Bauteils relativ zu der Faltkammer die Faltkammer von dem Deckel freizulegen und somit zu öffnen, sowie mittels einer Bewegung des zweiten Bauteils relativ zu der Faltkammer die Faltkammer zu deformieren und dadurch die Intraokularlinse zu falten.

Bei den beiden Ausführungsformen ist die Intraokularlinse zusammen mit der Aufbewahrungsflüssigkeit in der Faltkammer angeordnet, wobei der Deckel ein Ausfließen der Aufbewahrungsflüssigkeit in den ersten Zylinderabschnitt unterbindet. Dadurch kann dieser trocken gelagert werden, so dass eine in dem ersten Zylinderabschnitt vorgesehene Beschichtung, die ein Gleiten der Intraokularlinse in dem Injektor erleichtert, eine zeitlich lange Lagerstabilität hat.

Weil das Einsetzbauteil, in dem die Intraokularlinse angeordnet ist, bereits in der Aufnahme angeordnet ist, kann der gesamte Injektor einschließlich der Intraokularlinse sterilisiert werden und anschließend in einer einzelnen Verpackung geliefert werden. Ein Einsetzen der Intraokularlinse in den Injektor ist nicht mehr erforderlich, so dass das Risiko einer Kontamination im Vergleich zu einem herkömmlichen Injektor vermindert ist. Weil bei der ersten Ausführungsform durch die Bewegung des Gehäuses relativ zu der Faltkammer und bei der zweiten Ausführungsform durch die Bewegung des ersten Bauteils relativ zu der Faltkammer die Faltkammer geöffnet wird, ist es bei den beiden Ausführungsformen nicht erforderlich, den Deckel von Hand anzufassen, um den Deckel zu entfernen. Zudem handelt es sich gemäß der ersten Ausführungsform bei dem Gehäuse im Vergleich zu dem Deckel um ein eher großes Bauteil, was von Hand wesentlich einfacher als der Deckel angefasst werden kann und damit einfacher bedienbar ist. Außerdem erfüllt die Faltkammer bei den beiden Ausführungsformen zwei Funktionen, nämlich zum einen wird in ihr die Intraokularlinse vor dem Betrieb des Injektors gelagert und zum anderen wird während des Betriebs des Injektors mittels der Faltkammer die Intraokularlinse gefaltet. Gemäß der ersten Ausführungsform kann die Faltkammer mittels einer einzelnen Bewegung des Gehäuses relativ zu der Faltkammer geöffnet und deformiert werden. Gemäß der zweiten Ausführungsform kann die Faltkammer nacheinander mittels zweier Bewegungen, nämlich der Bewegung des ersten Bauteils relativ zu der Faltkammer und der Bewegung des zweiten Bauteils relativ zu der Faltkammer, zuerst geöffnet und anschließend deformiert oder zuerst deformiert und anschließend geöffnet werden.

Es ist für die beiden Ausführungsformen bevorzugt, dass der Injektor eine Kanüle aufweist und dass der erste Zylinderabschnitt zwischen der Kanüle und der Faltkammer angeordnet ist. Weil die Intraokularlinse bei dem Betrieb des Injektors auf ihrem Weg zu der Kanüle den ersten Zylinderabschnitt passiert, ist die Beschichtung dort vorgesehen. Damit ist es besonders relevant, mit dem Deckel die Faltkammer gegenüber dem ersten Zylinderabschnitt abzudichten, um einen Kontakt der Beschichtung mit der Aufbewahrungsflüssigkeit zu unterbinden.

Es ist für die beiden Ausführungsformen bevorzugt, dass das Einsetzbauteil einen weiteren Deckel aufweist, der die Faltkammer gegenüber den zweiten Zylinderabschnitt abdichtet. Besonders bevorzugt funktioniert der weitere Deckel sonst wie der Deckel.

Für die erste Ausführungsform ist es bevorzugt, dass der Injektor eine Einrastvorrichtung aufweist, die eingerichtet ist, die Position der Faltkammer in der Aufnahme festzulegen. Dadurch kann die Position der Faltkammer relativ zu dem Injektor auch so festgelegt werden, dass der Deckel fluchtend mit dem ersten Zylinderabschnitt und dem zweiten Zylinderabschnitt angeordnet ist, so dass in dem Betrieb des Injektors ein Kolben des Injektors in dem ersten Zylinderabschnitt, in dem zweiten Zylinderabschnitt und in der Faltkammer gleiten kann.

Es ist für die erste Ausführungsform erfindungsgemäß, dass das Gehäuse eine äußere Gehäusekontaktfläche aufweist und die Aufnahme eine Aufnahmekontaktfläche aufweist, wobei die äußere Gehäusekontaktfläche und die Aufnahmekontaktfläche aneinander anliegen und deren Normalen einen von 90° verschiedenen ersten Winkel mit einer Einschieberichtung des Einsetzbauteils einschließen, in der das Einsetzbauteil zum Zusammenbauen des Injektors in die Aufnahme einzuführen ist. Mittels der äußeren Gehäusekontaktfläche und der Aufnahmekontaktfläche ist die Position des Einsetzbauteils festgelegt und zum Fertigstellen des Injektors ist das Einsetzbauteil so weit in die Aufnahme einzuführen, bis die äußere Gehäusekontaktfläche und die Aufnahmekontaktfläche sich kontaktieren. In dem Fall, dass die Einrastvorrichtung vorgesehen ist, ist es bevorzugt, dass die Einrastvorrichtung einrastet und somit die Position der Faltkammer in der Aufnahme festlegt, sobald die äußere Gehäusekontaktfläche und die Aufnahmekontaktfläche sich kontaktieren. Zum Ausführen der Bewegung des Gehäuses relativ zu der Faltkammer ist das Gehäuse, insbesondere von Hand, entgegen die Einschieberichtung zu bewegen. Besonders bevorzugt beträgt der erste Winkel von 0° bis 60°.

Für die erste Ausführungsform ist es bevorzugt, dass das Gehäuse eine innere Gehäusekontaktfläche aufweist, die eingerichtet ist, die Faltkammer zu kontaktieren und zu deformieren, wenn die Bewegung des Gehäuses relativ zu der Faltkammer durchgeführt wird. Besonders bevorzugt schließt die Normale der inneren Gehäusekontaktfläche einen von 90° verschiedenen zweiten Winkel mit einer Einschieberichtung des Einsetzbauteils ein. Ganz besonders bevorzugt beträgt der zweite Winkel von 0° bis 60°. Besonders bevorzugt weist das Gehäuse zwei der inneren Gehäusekontaktflächen auf, die an einander zugewandten Wänden des Gehäuses angeordnet sind und spiegelsymmetrisch zueinander angeordnet sind. Damit ist es vorteilhaft möglich, die Faltkammer symmetrisch zu deformieren.

Es ist für die erste Ausführungsform bevorzugt, dass das Einsetzbauteil eingerichtet ist, dass bei der Bewegung des Gehäuses relativ zu der Faltkammer der Deckel an dem Gehäuse befestigt bleibt, um somit die Faltkammer zu öffnen. Dabei handelt es sich um eine vorteilhaft einfache Konstruktion, um die Faltkammer zu öffnen.

Es ist für die zweite Ausführungsform erfindungsgemäß, dass das erste Bauteil ein Gehäuse ist, innerhalb dessen die Faltkammer angeordnet ist. Das Gehäuse hat somit eine Doppelfunktion, nämlich zum einen haust es die Faltkammer ein, zum anderen trägt es den Deckel. Es ist besonders bevorzugt, dass das Gehäuse einen Griff aufweist. Dadurch kann das Gehäuse einfacher von Hand angefasst werden und somit kann ein Bediener des Injektors einfacher die Bewegung des ersten Bauteils relativ zu der Faltkammer durchführen als es ohne den Griff der Fall wäre.

Für die zweite Ausführungsform ist es bevorzugt, dass das Einsetzbauteil eingerichtet ist, dass bei der Bewegung des ersten Bauteils relativ zu der Faltkammer der Deckel an dem ersten Bauteil befestigt bleibt, um somit die Faltkammer zu öffnen. Dabei handelt es sich um eine vorteilhaft einfache Konstruktion, um die Faltkammer zu öffnen.

Es ist für die zweite Ausführungsform bevorzugt, dass das zweite Bauteil ein Schieber ist, der eingerichtet ist, die Faltkammer zu kontaktieren und zu deformieren, wenn die Bewegung des Schiebers relativ zu der Faltkammer durchgeführt wird. Der Schieber ist besonders bevorzugt längsverschiebbar an der Aufnahme gelagert.

Für die zweite Ausführungsform ist es bevorzugt, dass der Injektor eine Einrastvorrichtung aufweist, die eingerichtet ist, die Position der Faltkammer in der Aufnahme festzulegen. Dadurch kann die Position der Faltkammer relativ zu dem Injektor auch so festgelegt werden, dass der Deckel fluchtend mit dem ersten Zylinderabschnitt und dem zweiten Zylinderabschnitt angeordnet ist, so dass in dem Betrieb des Injektors ein Kolben des Injektors in dem ersten Zylinderabschnitt, in dem zweiten Zylinderabschnitt und in der Faltkammer gleiten kann.

Für die beiden Ausführungsformen ist es bevorzugt, dass die Aufbewahrungsflüssigkeit Wasser aufweist. Es ist besonders bevorzugt, dass die Aufbewahrungsflüssigkeit im Wesentlichen aus Wasser besteht oder eine wässrige Salzlösung mit einer isotonischen Salzkonzentration ist. Die wässrige Salzlösung kann auch einen Puffer aufweisen.

Für beide Ausführungsformen weist das erfindungsgemäße Verfahren zum Herstellen des Injektors folgende Schritte auf:
a) Herstellen des Einsetzbauteils;
b) Herstellen des Injektors ohne das Einsetzbauteil;
d) Einführen des Einsetzbauteils in die Aufnahme;
e) Sterilisieren des Injektors mit dem in die Aufnahme eingeführten Einsetzbauteil.

Es ist bevorzugt, dass das Verfahren vor dem Schritt d) den Schritt c) aufweist:
c) Sterilisieren des Einsetzbauteils und des Injektors ohne das Einsetzbauteil, insbesondere wobei das Sterilisieren ein Dampfsterilisieren ist.

Bevorzugt werden die Schritte a) und b) unter Reinraumbedingungen durchgeführt.

Es ist bevorzugt, dass in Schritt e) mittels Gammastrahlung und/oder Ethylenoxid sterilisiert wird.

Im Folgenden werden zwei bevorzugte Ausführungsformen des erfindungsgemäßen Injektors anhand der beigefügten schematischen Zeichnungen erläutert.
Figur 1 zeigt ein Einsetzbauteil einer ersten Ausführungsform des Injektors in einem Aufbewahrungszustand des Injektors.
Figur 2 zeigt das Einsetzbauteil aus Figur 1 in einem Betriebszustand des Injektors.
Figur 3 zeigt die erste Ausführungsform des Injektors in dem Aufbewahrungszustand.
Figur 4 zeigt die erste Ausführungsform des Injektors in dem Betriebszustand.
Figur 5 zeigt eine zweite Ausführungsform des Injektors in einem Aufbewahrungszustand des Injektors.
Figur 6 zeigt einen ersten Ausschnitt des Injektors in dem Aufbewahrungszustand.
Figur 7 zeigt den Ausschnitt aus Figur 6 in einem Betriebszustand des Injektors.
Figur 8 zeigt einen zweiten Ausschnitt des Injektors mit einer undeformierten Faltkammer.
Figur 9 zeigt den Ausschnitt aus Figur 8 mit einer deformierten Faltkammer.

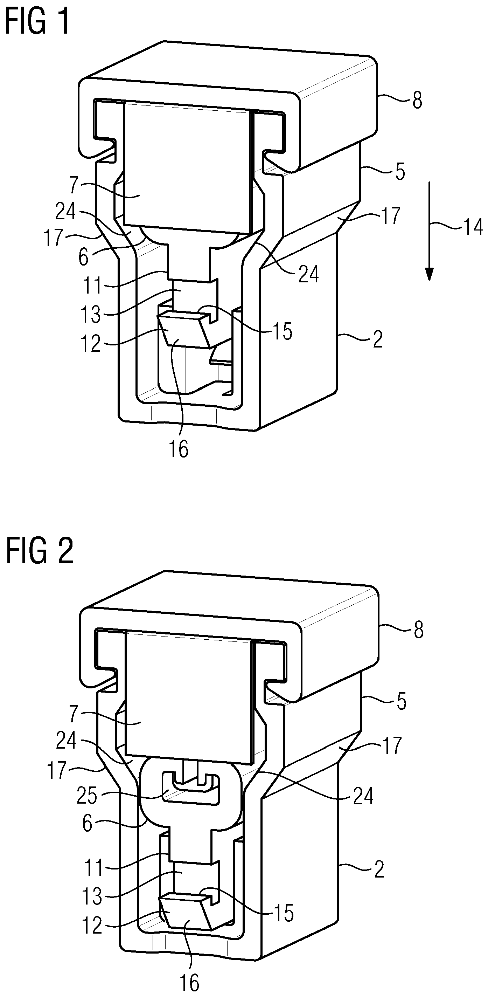

Wie es aus Figuren 1 bis 4 ersichtlich ist, weist gemäß der ersten Ausführungsform ein Injektor 1 einen Zylinder 3, eine Aufnahme 4 und ein Einsetzbauteil 2 auf. Der Zylinder 3 weist einen ersten Zylinderabschnitt 21 und einen zweiten Zylinderabschnitt 22 auf, die in Längsrichtung des Zylinders 3 nebeneinander angeordnet sind, und die Aufnahme 4 ist zwischen den beiden Zylinderabschnitten 21, 22 angeordnet. Das Einsetzbauteil 2 ist in der Aufnahme 4 angeordnet. Zudem weist das Einsetzbauteil 2 eine Intraokularlinse, eine Aufbewahrungsflüssigkeit, ein Gehäuse 5, eine innerhalb des Gehäuses 5 angeordnete Faltkammer 6, in der die Intraokularlinse von der Aufbewahrungsflüssigkeit umgeben angeordnet ist, und einen Deckel 7 auf. Der Deckel 7 dichtet die Faltkammer 6 gegenüber dem ersten Zylinderabschnitt 21 ab und ist an dem Gehäuse 5 befestigt. Das Einsetzbauteil 2 ist eingerichtet, mittels einer Bewegung des Gehäuses 5 relativ zu der Faltkammer 6 die Faltkammer 6 von dem Deckel 7 freizulegen und somit zu öffnen sowie die Faltkammer 6 zu deformieren und dadurch die Intraokularlinse zu falten.

Das Einsetzbauteil 2 kann einen weiteren Deckel aufweisen, der die Faltkammer 6 gegenüber dem zweiten Zylinderabschnitt 22 abdichtet. Der weitere Deckel kann baugleich zu dem Deckel 7 sein und auch sonst wie der Deckel 7 funktionieren. Es ist denkbar, dass die Faltkammer 6 eine Öffnung 25 aufweist, die von dem Deckel 7 abgedichtet ist, und eine weitere Öffnung aufweist, die von dem weiteren Deckel abgedichtet ist.

Der Injektor 1 kann eine Einrastvorrichtung aufweisen, die eingerichtet ist, die Position der Faltkammer 6 in der Aufnahme 4 festzulegen. Dabei ist denkbar, dass in der festgelegten Position der Faltkammer 6 die Öffnung 25 und die weitere Öffnung fluchtend mit dem Zylinder 3 angeordnet sind, so dass ein Kolben des Injektors 1 in einem Betrieb des Injektors 1 sich in dem ersten Zylinderabschnitt 21, dem zweiten Zylinderabschnitt 22 und der Faltkammer 6 erstrecken kann und somit die Intraokularlinse mittels des Kolbens von der Faltkammer 6 bis in eine Kanüle 23 des Injektors 1 beförderbar ist.

Die Einrastvorrichtung kann, wie es auch in Figuren 1 und 2 gezeigt ist, eine Nut 13 und einen Vorsprung (nicht dargestellt) aufweisen, der zum Einrasten der Einrastvorrichtung in die Nut 13 eingreift. Eines der Nut 13 und des Vorsprungs ist an der Faltkammer 6 angeordnet und das andere der Nut 13 und des Vorsprungs ist an der Aufnahme 4 angeordnet. Die Faltkammer 6 kann einen nach außen bezüglich der Faltkammer 6 abstehenden Faltkammerzapfen 11 aufweisen, an dem das eine der Nut 13 und des Vorsprungs angeordnet ist. Die Einrastvorrichtung kann zudem einen Nutbegrenzungsvorsprung 12 aufweisen, der eine erste Flanke 15 aufweist, die die Nut 13 begrenzt, und eine zweite Flanke 16 aufweist, die mit der ersten Flanke 15 einen Winkel kleiner als 90° einschließt, insbesondere kleiner als 60°. Die Normale der ersten Flanke 15 kann parallel zu der Einschieberichtung 14 angeordnet sein. Bei Einführen des Einsetzbauteils 2 in die Aufnahme 4 kann dadurch der Vorsprung auf der zweiten Flanke 16 leicht gleiten, und sobald der Vorsprung die zweite Flanke 16 passiert hat, greift er in die Nut 13 ein, wodurch die Einrastvorrichtung einrastet.

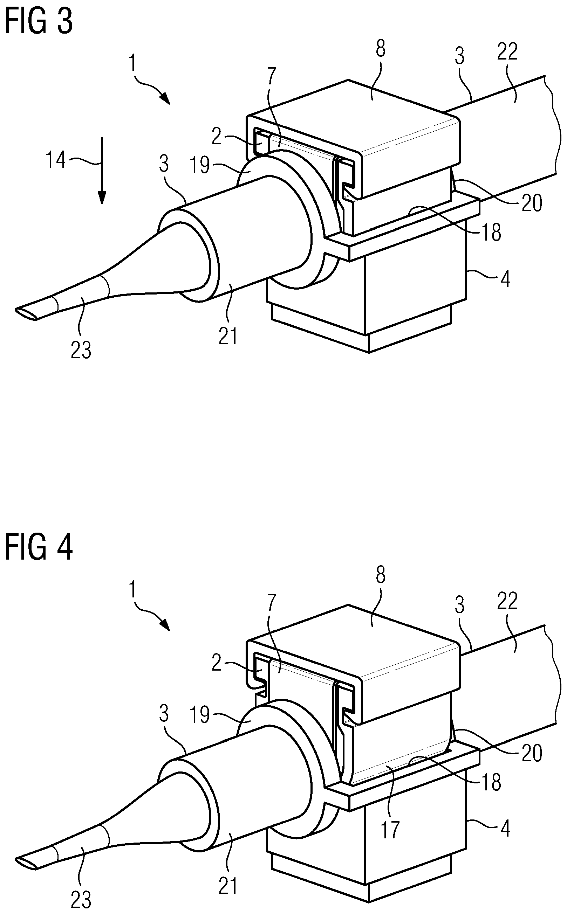

Wie in Figuren 3 und 4 dargestellt, weist das Gehäuse 5 eine äußere Gehäusekontaktfläche 17 auf und die Aufnahme 4 weist eine Aufnahmekontaktfläche 18 auf, wobei die äußere Gehäusekontaktfläche 17 und die Aufnahmekontaktfläche 18 aneinander anliegen und deren Normalen einen von 90° verschiedenen ersten Winkel mit einer Einschieberichtung 14 des Einsetzbauteils 2 einschließen, in der das Einsetzbauteil 2 zum Zusammenbauen des Injektors 1 in die Aufnahme 4 einzuführen ist. Wenn die äußere Gehäusekontaktfläche 17 und die Aufnahmekontaktfläche 18 aneinander anliegen, befindet sich der Injektor 1 in einem Aufbewahrungszustand, der in Figur 3 dargestellt ist. Es ist denkbar, dass die Einrastvorrichtung einrastet und somit die Position der Faltkammer 6 in der Aufnahme 4 festlegt, sobald beim Einführen des Einsetzbauteils 2 in die Aufnahme 4 die äußere Gehäusekontaktfläche 17 und die Aufnahmekontaktfläche 18 aneinander anliegen und der Injektor 1 sich somit in dem Aufbewahrungszustand befindet.

Figuren 1 bis 4 zeigen, dass das Einsetzbauteil 2 eingerichtet sein kann, dass bei der Bewegung des Gehäuses 5 relativ zu der Faltkammer 6 der Deckel 7 an dem Gehäuse 5 befestigt bleibt. Somit ist das Einsetzbauteil 2 eingerichtet, die Faltkammer 6 zu öffnen. Um den Injektor 1 in einen Betriebszustand des Injektors 1 zu bringen, ist das Gehäuse 5 aus dem Aufbewahrungszustand entgegen der Einschieberichtung 14 zu bewegen, wie es in Figur 4 dargestellt ist. Figur 1 zeigt den Einsetzkörper 2 in dem Aufbewahrungszustand und Figur 2 zeigt den Einsetzkörper 2 in dem Betriebszustand. In Figur 1 ist ersichtlich, dass der Deckel 7 die Faltkammer 6 in dem Aufbewahrungszustand abdichtet. Figur 2 zeigt, dass in dem Betriebszustand die Faltkammer 6 von dem Deckel 7 freigelegt ist, weil der Deckel 7 an dem Gehäuse 5 befestigt bleibt. Zum Befestigen des Deckels 5 an dem Gehäuse 5 kann, wie in Figuren 1 bis 4 dargestellt, das Gehäuse 5 eine Kappe 8 aufweisen, die an dem verbliebenen Gehäuse 5 befestigt ist, und der Deckel 7 zwischen der Kappe 8 und dem verbliebenen Gehäuse 5 eingeklemmt sein.

Figuren 1 und 2 zeigen, dass das Gehäuse 5 eine innere Gehäusekontaktfläche 24 aufweisen kann, die eingerichtet ist, die Faltkammer 6 zu kontaktieren und zu deformieren, wenn die Bewegung des Gehäuses 5 relativ zu der Faltkammer 6 durchgeführt wird. Dabei kann die Normale der inneren Gehäusekontaktfläche 24 einen von 90° verschiedenen zweiten Winkel mit einer Einschieberichtung 14 des Einsetzbauteils 2 einschließen, in der das Einsetzbauteil 2 in die Aufnahme 4 einzuführen ist. Insbesondere beträgt der zweite Winkel von 0° bis 60°. Zudem kann das Gehäuse 5 zwei der inneren Gehäusekontaktflächen 24 aufweisen, die an einander zugewandten Wänden des Gehäuses angeordnet sind und spiegelsymmetrisch zueinander angeordnet sind. Die Faltkammer 6 kann ein flexibles Material aufweisen. Auch ist es denkbar, dass die Faltkammer 6 Sollbruchstellen aufweist, die eingerichtet sind zu brechen, während die Faltkammer 6 deformiert wird.

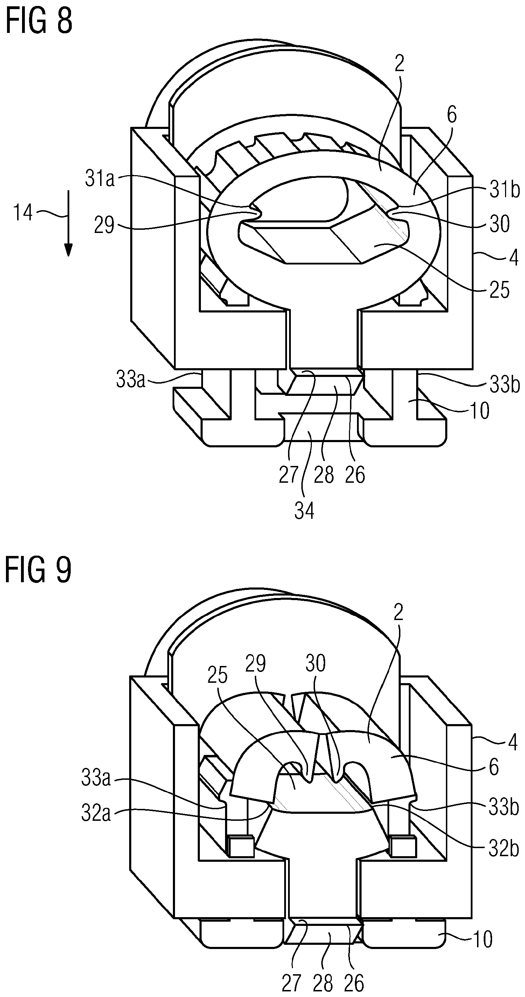

Die Faltkammer 6 kann aufgebaut sein, wie sie in Figuren 8 und 9 dargestellt ist. Dort ist dargestellt, dass die Faltkammer 6 beispielsweise einen ovalen Querschnitt haben kann. Die Faltkammer 6 weist einen ersten Faltkammervorsprung 29 und einen zweiten Faltkammervorsprung 30 auf, die in das Innere der Faltkammer 6 ragen und beabstandet zueinander angeordnet sind. Unmittelbar benachbart zu dem ersten Faltkammervorsprung 29 und dem zweiten Faltkammervorsprung 30 ist jeweils eine Sollbruchstelle 31a, 31b angeordnet, so dass bei dem Deformieren der Faltkammer 6 der Abschnitt der Faltkammer **6,** der zwischen dem ersten Faltkammervorsprung 29 und dem zweiten Faltkammervorsprung 30 angeordnet ist, von dem verbliebenen Teil der Faltkammer 6 abgetrennt wird. Zudem kontaktieren sich der erste Faltkammervorsprung 29 und der zweite Faltkammervorsprung 30 nach dem Deformieren. Die Faltkammer 6 kann in dem verbliebenen Teil weitere Sollbruchstellen 32a, 32b aufweisen, die ein stärkeres Deformieren der Faltkammer 6 erlauben, als es ohne die weiteren Sollbruchstellen 32a, 32b der Fall wäre.

Wie es aus Figuren 3 und 4 ersichtlich ist, kann die Aufnahme 4 vier Wände aufweisen, die in der Draufsicht in Form eines Rechtecks angeordnet sind und innerhalb derer das Einsetzbauteil 2 angeordnet ist. Zudem kann der erste Zylinderabschnitt 21 einen ersten Zylindervorsprung 19 aufweisen, der an dem dem Einsetzkörper 2 zugewandten Ende des ersten Zylinderabschnitts 21 angeordnet ist und von dem ersten Zylinderabschnitt 21 radial nach außen absteht. Analog kann der zweite Zylinderabschnitt 22 einen zweiten Zylindervorsprung 20 aufweisen, der an dem dem Einsetzkörper 2 zugewandten Ende des zweiten Zylinderabschnitts 22 angeordnet ist und von dem ersten Zylinderabschnitt 22 radial nach außen absteht. Der erste Zylindervorsprung 19 und der zweite Zylindervorsprung 20 stützen das Einsetzbauteil 2 in Längsrichtung des Zylinders 3 zusätzlich ab.

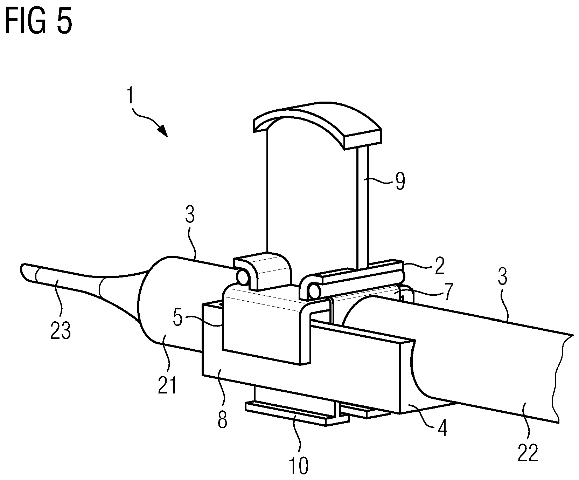

Wie es aus Figuren 5 bis 7 ersichtlich ist, weist gemäß der zweiten Ausführungsform ein Injektor 1 einen Zylinder 3, eine Aufnahme 4, ein Einsetzbauteil 2 und ein zweites Bauteil auf. Der Zylinder 3 weist einen ersten Zylinderabschnitt 21 und einen zweiten Zylinderabschnitt 22 auf, die in Längsrichtung des Zylinders 3 nebeneinander angeordnet sind. Die Aufnahme 4 ist zwischen den beiden Zylinderabschnitten 21, 22 angeordnet. Das Einsetzbauteil 2 ist in der Aufnahme 4 angeordnet und weist eine Intraokularlinse, eine Aufbewahrungsflüssigkeit, ein Gehäuse 5, eine innerhalb des Gehäuses 5 angeordnete Faltkammer 6, in der die Intraokularlinse von der Aufbewahrungsflüssigkeit umgeben angeordnet ist, einen Deckel 7, der die Faltkammer 6 gegenüber dem ersten Zylinderabschnitt 21 abdichtet, und ein erstes Bauteil auf, an dem der Deckel 7 angeordnet ist. Der Injektor 1 ist eingerichtet, mittels einer Bewegung des ersten Bauteils relativ zu der Faltkammer 6 die Faltkammer 6 von dem Deckel 7 freizulegen und somit zu öffnen, sowie mittels einer Bewegung des zweiten Bauteils relativ zu der Faltkammer 6 die Faltkammer 6 zu deformieren und dadurch die Intraokularlinse zu falten.

Das Einsetzbauteil kann einen weiteren Deckel aufweisen, der die Faltkammer gegenüber dem zweiten Zylinderabschnitt 22 abdichtet. Der weitere Deckel kann baugleich zu dem Deckel 7 sein und auch sonst wie der Deckel 7 funktionieren. Es ist denkbar, dass die Faltkammer 6 eine Öffnung 25 aufweist, die von dem Deckel 7 abgedichtet ist, und eine weitere Öffnung aufweist, die von dem weiteren Deckel abgedichtet ist.

Der Injektor 1 kann eine Einrastvorrichtung aufweisen, die eingerichtet ist, die Position der Faltkammer 6 in der Aufnahme 4 festzulegen. Dabei ist denkbar, dass in der festgelegten Position der Faltkammer 6 die Öffnung 25 und die weitere Öffnung fluchtend mit dem Zylinder 3 angeordnet sind, so dass ein Kolben des Injektors 1 in einem Betrieb des Injektors sich in dem ersten Zylinderabschnitt 21, dem zweiten Zylinderabschnitt 22 und der Faltkammer 6 erstrecken kann und somit die Intraokularlinse mittels des Kolbens von der Faltkammer 6 bis in eine Kanüle 23 des Injektors 1 beförderbar ist.

Die Einrastvorrichtung kann, wie es auch in Figuren 8 und 9 gezeigt ist, eine Aussparung (nicht dargestellt) und einen Einrastvorsprung 26 aufweisen, der zum Einrasten der Einrastvorrichtung in die Aussparung eingreift. Eines des Einrastvorsprungs 26 und der Aussparung ist an der Faltkammer 6 angeordnet und das andere des Einrastvorsprungs 26 und der Aussparung ist an der Aufnahme 4 angeordnet. Die Faltkammer 6 kann einen nach außen bezüglich der Faltkammer 6 abstehenden Faltkammerzapfen 11 aufweisen, an dem das eine des Einrastvorsprungs 26 und der Aussparung angeordnet ist. Der Einrastvorsprung 26 kann eine erste Flanke 27, deren Normale im Wesentlichen parallel zu der Einschieberichtung 14 ist, und eine zweite Flanke 28 aufweisen, die mit der ersten Flanke 27 einen Winkel kleiner als 90° einschließt, insbesondere kleiner als 60°. Bei Einführen des Einsetzbauteils 2 in die Aufnahme 4 kann dadurch der an die Aussparung angrenzende Bereich leicht auf der zweiten Flanke 28 gleiten und sobald der an die Aussparung angrenzende Bereich die zweite Flanke 28 passiert hat, greift der Einrastvorsprung 26 in die Aussparung ein, wodurch die Einrastvorrichtung einrastet.

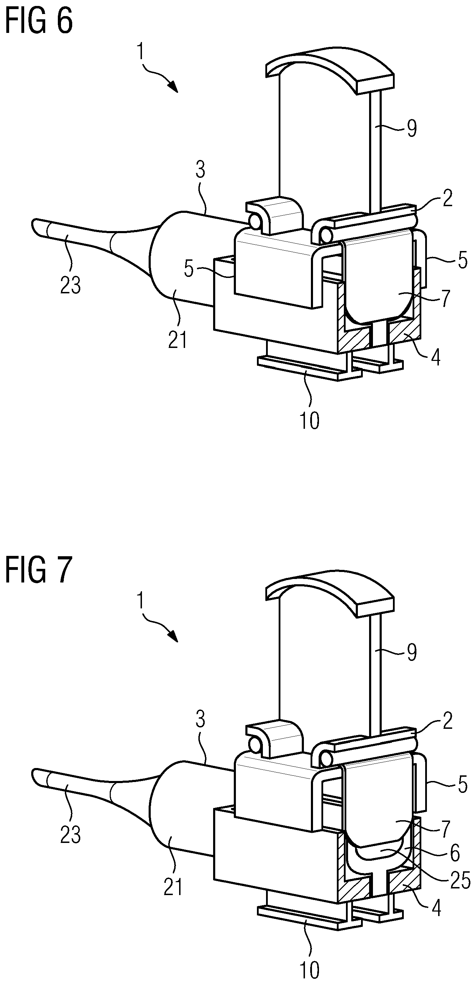

Figuren 5 bis 7 zeigen, dass das erste Bauteil ein Gehäuse 5 ist, innerhalb dessen die Faltkammer 6 angeordnet ist. Das Gehäuse 5 kann längsverschiebbar in der Aufnahme 4 angeordnet sein. Zudem kann das Gehäuse 5 einen Griff 9 aufweisen, der von dem Gehäuse 5 in Richtung von der Aufnahme 4 weg von dem Gehäuse 5 absteht. Figur 5 zeigt einen Aufbewahrungszustand des Injektors, in dem der Deckel 7 die Faltkammer 6 abdichtet. Das Einsetzbauteil 2 ist eingerichtet, dass bei der Bewegung des ersten Bauteils relativ zu der Faltkammer 6 der Deckel 7 an dem Gehäuse 5 befestigt bleibt, um somit die Faltkammer 6 zu öffnen. Figur 7 zeigt den Fall, dass durch die Bewegung des Gehäuses 5 relativ zu der Faltkammer 6 der Injektor 1 von dem Aufbewahrungszustand in einen Betriebszustand des Injektors 1 gebracht wird und dadurch die Faltkammer 6 von dem Deckel 7 freigelegt wird.

Wie es aus Figuren 8 und 9 ersichtlich ist, kann das zweite Bauteil ein Schieber 10 sein, der eingerichtet ist, die Faltkammer 6 zu kontaktieren und zu deformieren, wenn die Bewegung des Schiebers 10 relativ zu der Faltkammer 6 durchgeführt wird. Dabei kann der Schieber 10 parallel zur Einschieberichtung 14 längsverschiebbar an der Aufnahme 4 gelagert sein. Zudem kann der Schieber 10 sich auch nach außerhalb der Aufnahme 4 erstrecken, damit der Schieber 10 von außerhalb der Aufnahme 4 von Hand gedrückt werden kann. Der Schieber 10 kann einen ersten Arm 33a, einen zweiten Arm 33b und einen Steg 34 aufweisen, der den ersten Arm 33a mit dem zweiten Arm 33b verbindet. Der erste Arm 33a und der zweite Arm 33b sind jeweils in einem Bereich gegenüberliegender Enden der Faltkammer 6 angeordnet, so dass der Schieber 10 eingerichtet ist, die Faltkammer 6 symmetrisch zu deformieren.

Die Faltkammer 6 kann ein flexibles Material aufweisen. Auch ist es denkbar, dass die Faltkammer 6 Sollbruchstellen aufweist, die eingerichtet sind zu brechen, während die Faltkammer 6 deformiert wird. In Figuren 8 und 9 ist dargestellt, dass die Faltkammer 6 beispielsweise einen ovalen Querschnitt haben kann. Die Faltkammer 6 weist einen ersten Faltkammervorsprung 29 und einen zweiten Faltkammervorsprung 30 auf, die in das Innere der Faltkammer 6 ragen und beabstandet zueinander angeordnet sind. Unmittelbar benachbart zu dem ersten Faltkammervorsprung 29 und dem zweiten Faltkammervorsprung 30 ist jeweils eine Sollbruchstelle 31a, 31b angeordnet, so dass bei dem Deformieren der Faltkammer 6 der Abschnitt der Faltkammer 6, der zwischen dem ersten Faltkammervorsprung 29 und dem zweiten Faltkammervorsprung 30 angeordnet ist, von dem verbliebenen Teil der Faltkammer 6 abgetrennt wird. Zudem kontaktieren sich der erste Faltkammervorsprung 29 und der zweite Faltkammervorsprung 30 nach dem Deformieren. Die Faltkammer 6 kann in dem verbliebenen Teil weitere Sollbruchstellen 32a, 32b aufweisen, die ein stärkeres Deformieren der Faltkammer 6 erlauben, als es ohne die weiteren Sollbruchstellen 32a, 32b der Fall wäre.

### Bezugszeichenliste

1 Injektor
2 Einsetzbauteil
3 Zylinder
4 Aufnahme
5 Gehäuse
6 Faltkammer
7 Deckel
8 Kappe
9 Griff
10 Schieber
11 Faltkammerzapfen
12 Nutbegrenzungsvorsprung
13 Nut
14 Einschieberichtung
15 erste Flanke
16 zweite Flanke
17 äußere Gehäusekontaktfläche
18 Aufnahmekontaktfläche
19 erster Zylindervorsprung
20 zweiter Zylindervorsprung
21 erster Zylinderabschnitt
22 zweiter Zylinderabschnitt
23 Kanüle
24 innere Gehäusekontaktfläche
25 Öffnung
26 Einrastvorsprung
27 erste Flanke
28 zweite Flanke
29 erster Faltkammervorsprung
30 zweiter Faltkammervorsprung
31a Sollbruchstelle
31b Sollbruchstelle
32a weitere Sollbruchstelle
32b weitere Sollbruchstelle
33a erster Arm
33b zweiter Arm
34 Steg

## Patentansprüche

1. Injektor mit einem Zylinder (3), der einen ersten Zylinderabschnitt (21) und einen zweiten Zylinderabschnitt (22) aufweist, die in Längsrichtung des Zylinders (3) nebeneinander angeordnet sind, einer Aufnahme (4), die zwischen den beiden Zylinderabschnitten (21, 22) angeordnet ist, und einem Einsetzbauteil (2), das in der Aufnahme (4) angeordnet ist, das eine Intraokularlinse, eine Aufbewahrungsflüssigkeit, ein Gehäuse (5), eine innerhalb des Gehäuses (5) angeordnete Faltkammer (6), in der die Intraokularlinse von der Aufbewahrungsflüssigkeit umgeben angeordnet ist, und einen Deckel (7) aufweist, der die Faltkammer (6) gegenüber einem des ersten Zylinderabschnitts (21) und des zweiten Zylinderabschnitts (22) abdichtet sowie an dem Gehäuse (5) befestigt ist, wobei das Einsetzbauteil (2) eingerichtet ist, mittels einer Bewegung des Gehäuses (5) relativ zu der Faltkammer (6) die Faltkammer (6) von dem Deckel (7) freizulegen und somit zu öffnen sowie die Faltkammer (6) zu deformieren und dadurch die Intraokularlinse zu falten, **dadurch gekennzeichnet, dass** das Gehäuse (5) eine äußere Gehäusekontaktfläche (17) aufweist und die Aufnahme (4) eine Aufnahmekontaktfläche (18) aufweist, wobei die Gehäusekontaktfläche (17) und die Aufnahmekontaktfläche (18) aneinander anliegen und deren Normalen einen von 90° verschiedenen ersten Winkel mit einer Einschieberichtung (14) des Einsetzbauteils (2) einschließen, in der das Einsetzbauteil (2) zum Zusammenbauen des Injektors (1) in die Aufnahme (4) einzuführen ist.

2. Injektor gemäß Anspruch 1, wobei das Gehäuse (5) eine innere Gehäusekontaktfläche (24) aufweist, die eingerichtet ist, die Faltkammer (6) zu kontaktieren und zu deformieren, wenn die Bewegung des Gehäuses (5) relativ zu der Faltkammer (6) durchgeführt wird.

3. Injektor gemäß Anspruch 1 oder 2, wobei das Einsetzbauteil (2) eingerichtet ist, dass bei der Bewegung des Gehäuses (5) relativ zu der Faltkammer (6) der Deckel (7) an dem Gehäuse (5) befestigt bleibt, um somit die Faltkammer (6) zu öffnen.

4. Injektor mit einem Zylinder (3), der einen ersten Zylinderabschnitt (21) und einen zweiten Zylinderabschnitt (22) aufweist, die in Längsrichtung des Zylinders (3) nebeneinander angeordnet sind, einer Aufnahme (4), die zwischen den beiden Zylinderabschnitten (21, 22) angeordnet ist, einem Einsetzbauteil (2), das in der Aufnahme (4) angeordnet ist, das eine Intraokularlinse, eine Aufbewahrungsflüssigkeit, ein Gehäuse (5), eine innerhalb des Gehäuses (5) angeordnete Faltkammer (6), in der die Intraokularlinse von der Aufbewahrungsflüssigkeit umgeben angeordnet ist, einen Deckel (7), der die Faltkammer (6) gegenüber einem des ersten Zylinderabschnitts (21) und des zweiten Zylinderabschnitts (22) abdichtet, und ein erstes Bauteil aufweist, an dem der Deckel (7) angeordnet ist, und einem zweiten Bauteil, wobei der Injektor (1) eingerichtet ist, mittels einer Bewegung des ersten Bauteils relativ zu der Faltkammer (6) die Faltkammer (6) von dem Deckel (7) freizulegen und somit zu öffnen, sowie mittels einer Bewegung des zweiten Bauteils relativ zu der Faltkammer (6) die Faltkammer (6) zu deformieren und dadurch die Intraokularlinse zu falten, **dadurch gekennzeichnet, dass** das erste Bauteil das Gehäuse (5) ist, innerhalb dessen die Faltkammer (6) angeordnet ist.

5. Injektor gemäß Anspruch 4, wobei das Einsetzbauteil (2) eingerichtet ist, dass bei der Bewegung des ersten Bauteils relativ zu der Faltkammer (6) der Deckel (7) an dem ersten Bauteil befestigt bleibt, um somit die Faltkammer (6) zu öffnen.

6. Injektor gemäß Anspruch 4 oder 5, wobei das zweite Bauteil ein Schieber (10) ist, der eingerichtet ist, die Faltkammer (6) zu kontaktieren und zu deformieren, wenn die Bewegung des Schiebers (10) relativ zu der Faltkammer (6) durchgeführt wird.

7. Injektor gemäß Anspruch 6, wobei der Schieber (10) längsverschiebbar an der Aufnahme (4) gelagert ist.

8. Injektor gemäß einem der Ansprüche 1 bis 7, wobei der Injektor (1) eine Einrastvorrichtung aufweist, die eingerichtet ist, die Position der Faltkammer (6) in der Aufnahme (4) festzulegen.

## Claims

1. Injector having a cylinder (3), which has a first cylinder portion (21) and a second cylinder portion (22), which are arranged next to each other in the longitudinal direction of the cylinder (3), a receptacle (4), which is arranged between the two cylinder portions (21, 22), and an insert component (2), which is arranged in the receptacle (4) and which has an intraocular lens, a storage liquid, a housing (5), a folding chamber (6) which is arranged within the housing (5) and in which the intraocular lens is arranged so as to be surrounded by the storage liquid, and a cover (7) which seals the folding chamber (6) with respect to one of the first cylinder portion (21) and the second cylinder portion (22) and is fastened to the housing (5), wherein the insert component (2) is configured, by means of a movement of the housing (5) relative to the folding chamber (6), to free the folding chamber (6) from the cover (7) and thereby open it, and to deform the folding chamber (6) and thereby fold the intraocular lens, **characterized in that** the housing (5) has an outer housing contact surface (17) and the receptacle (4) has a receptacle contact surface (18), wherein the housing contact surface (17) and the receptacle contact surface (18) bear against each other and their normals enclose a first angle different than 90° with respect to an insertion direction (14) of the insert component (2), in which direction the insert component (2) is to be inserted into the receptacle (4) in order to assemble the injector (1).

2. Injector according to Claim 1, wherein the housing (5) has an inner housing contact surface (24) which is configured to contact and deform the folding chamber (6) when the movement of the housing (5) relative to the folding chamber (6) is carried out.

3. Injector according to Claim 1 or 2, wherein the insert component (2) is configured such that, during the movement of the housing (5) relative to the folding chamber (6), the cover (7) remains fastened to the housing (5) in order thereby to open the folding chamber (6) .

4. Injector having a cylinder (3), which has a first cylinder portion (21) and a second cylinder portion (22), which are arranged next to each other in the longitudinal direction of the cylinder (3), a receptacle (4), which is arranged between the two cylinder portions (21, 22), an insert component (2), which is arranged in the receptacle (4) and which has an intraocular lens, a storage liquid, a housing (5), a folding chamber (6) which is arranged within the housing (5) and in which the intraocular lens is arranged so as to be surrounded by the storage liquid, a cover (7) which seals the folding chamber (6) with respect to one of the first cylinder portion (21) and the second cylinder portion (22), and a first component on which the cover (7) is arranged, and a second component, wherein the injector (1) is configured, by means of a movement of the first component relative to the folding chamber (6), to free the folding chamber (6) from the cover (7) and thereby open it, and, by means of a movement of the second component relative to the folding chamber (6), to deform the folding chamber (6) and thereby fold the intraocular lens, **characterized in that** the first component is the housing (5), within which the folding chamber (6) is arranged.

5. Injector according to Claim 4, wherein the insert component (2) is configured such that, during the movement of the first component relative to the folding chamber (6), the cover (7) remains fastened to the first component in order thereby to open the folding chamber (6) .

6. Injector according to Claim 4 or 5, wherein the second component is a slide (10) which is configured to contact and deform the folding chamber (6) when the movement of the slide (10) relative to the folding chamber (6) is carried out.

7. Injector according to Claim 6, wherein the slide (10) is mounted in a longitudinally displaceable manner on the receptacle (4).

8. Injector according to one of Claims 1 to 7, wherein the injector (1) has a latching device which is configured to fix the position of the folding chamber (6) in the receptacle (4).

## Revendications

1. Injecteur avec un cylindre (3), qui comporte une première section (21) de cylindre et une seconde section (22) de cylindre, qui sont disposées côte à côte dans le sens longitudinal du cylindre (3), un logement (4), qui est disposé entre les deux sections (21, 22) de cylindre, et un composant d'insertion (2), qui est disposé dans le logement (4), qui comporte une lentille intraoculaire, un liquide de conservation, un boîtier (5), une chambre de pliage (6) disposée à l'intérieur du boîtier (5), dans laquelle la lentille intraoculaire est disposée de manière entourée par le liquide de conservation, et un couvercle (7), qui étanchéifie la chambre de pliage (6) par rapport à une de la première section (21) de cylindre et de la seconde section (22) de cylindre et est fixé sur le boîtier (5), le composant d'insertion (2) étant mis au point pour dégager, au moyen d'un déplacement du boîtier (5) par rapport à la chambre de pliage (6), la chambre de pliage (6) du couvercle (7) et ainsi l'ouvrir et pour déformer la chambre de pliage (6) et plier ainsi la lentille intraoculaire, **caractérisé en ce que** le boîtier (5) comporte une surface de contact extérieure (17) de boîtier et le logement (4) comporte une surface de contact (18) de logement, la surface de contact (17) de boîtier et la surface de contact (18) de logement reposant l'une sur l'autre et leurs normales forment un premier angle différent de 90° avec une direction d'emboîtement (14) du composant d'insertion (2), dans laquelle le composant d'insertion (2) doit être introduit dans le logement (4) pour l'assemblage de l'injecteur (1).

2. Injecteur selon la revendication 1, le boîtier (5) comportant une surface de contact intérieure (24) de boîtier, qui est mise au point pour entrer en contact avec la chambre de pliage (6) et la déformer lorsque le déplacement du boîtier (5) est effectué par rapport à la chambre de pliage (6).

3. Injecteur selon la revendication 1 ou 2, le composant d'insertion (2) étant mis au point pour que le couvercle (7) reste fixé sur le boîtier (5) lors du déplacement du boîtier (5) par rapport à la chambre de pliage (6) pour ouvrir ainsi la chambre de pliage (6).

4. Injecteur avec un cylindre (3), qui comporte une première section (21) de cylindre et une seconde section (22) de cylindre, qui sont disposées côte à côte dans le sens longitudinal du cylindre (3), un logement (4), qui est disposé entre les deux sections (21, 22) de cylindre, un composant d'insertion (2), qui est disposé dans le logement (4), lequel comporte une lentille intraoculaire, un liquide de conservation, un boîtier (5), une chambre de pliage (6) disposée à l'intérieur du boîtier (5), dans laquelle la lentille intraoculaire est disposée de manière entourée par le liquide de conservation, un couvercle (7), qui étanchéifie la chambre de pliage (6) par rapport à une de la première section (21) de cylindre et de la seconde section (22) de cylindre, et un premier composant, sur lequel le couvercle (7) est disposé, et un deuxième composant, l'injecteur (1) étant mis au point pour dégager, au moyen d'un déplacement du premier composant par rapport à la chambre de pliage (6), la chambre de pliage (6) du couvercle (7) et l'ouvrir ainsi, et pour déformer la chambre de pliage (6) au moyen d'un déplacement du deuxième composant par rapport à la chambre de pliage (6) et pour plier ainsi la lentille intraoculaire, **caractérisé en ce que** le premier composant est le boîtier (5), à l'intérieur duquel la chambre de pliage (6) est disposée.

5. Injecteur selon la revendication 4, le composant d'insertion (2) étant mis au point pour que le couvercle (7) reste fixé sur le premier composant lors du déplacement du premier composant par rapport à la chambre de pliage (6) pour ouvrir ainsi la chambre de pliage (6).

6. Injecteur selon la revendication 4 ou 5, le deuxième composant comportant un coulisseau (10), qui est mis au point pour entrer en contact avec la chambre de pliage (6) et la déformer lorsque le déplacement du coulisseau (10) est effectué par rapport à la chambre de pliage (6).

7. Injecteur selon la revendication 6, le coulisseau (10) étant monté sur le logement (4) de manière à pouvoir être coulissé longitudinalement.

8. Injecteur selon l'une des revendications 1 à 7, l'injecteur (1) comportant un dispositif d'enclenchement, qui est mis au point pour fixer la position de la chambre de pliage (6) dans le logement (4).
